Europäisches Patentamt

European Patent Office

Office européen des brevets

(19)

(11) Publication number: **0 145 818**
**B1**

(12) EUROPEAN PATENT SPECIFICATION

(45) Date of publication of patent specification: **12.10.88**

(51) Int. Cl.⁴: **G 01 N 3/00**, G 01 N 33/38

(21) Application number: **83307732.4**

(22) Date of filing: **20.12.83**

(54) Device and procedure for measuring in situ strength of concrete and the like.

(43) Date of publication of application:
**26.06.85 Bulletin 85/26**

(45) Publication of the grant of the patent:
**12.10.88 Bulletin 88/41**

(84) Designated Contracting States:
**DE FR GB**

(56) References cited:
**WO-A-82/02949**
**US-A-3 640 126**
**US-A-4 103 540**

**PATENTS ABSTRACTS OF JAPAN, vol. 6, no.
46 (P-107)924r, 24th March 1982; & JP - A - 56
160 636 (SHIMIZU KENSETSU K.K.) 10-12-1981**

(73) Proprietor: **Stoll, Ulrich W.**
**2121 Hall**
**Ann Arbor Michigan 48104 (US)**

(72) Inventor: **Stoll, Ulrich W.**
**2121 Hall**
**Ann Arbor Michigan 48104 (US)**

(74) Representative: **Williams, Trevor John et al
J.A. KEMP & CO. 14 South Square Gray's Inn
London WC1R 5EU (GB)**

## Description

In construction projects utilizing concrete structural members such as cast-in-place reinforced concrete beams or columns in a multi-storey structure, it is important to have the capability to measure in situ the compressive strength of particular structural members. In particular, it is often desirable to establish whether a lower limit strength has been achieved in critical members to assure that it is safe to remove temporary supplemental supports and/or impose, additional loading during construction. Delaying unnecessarily the removal of support or the adding of such additional loading will lengthen the construction period and correspondingly increase project costs.

A common method for estimating in situ the strength of concrete structural members is the "pull out" test. This test normally calls for an anchor bolt with an appropriately sized transverse flange or extension to be embedded in the concrete before hardening. After the concrete has hardened, the exposed end of the anchor bolt is pulled and the force required to rupture the concrete is measured. A hydraulic jack, screw jack or the like is typically employed to apply the necessary pulling force to the anchor bolt, and necessarily exerts a reacting force against the surface of the concrete structure.

Several limitations and deficiencies in the pull out test have become apparent. First, in order to limit the amount of required pull-out force and to preclude a non-indicative local crushing failure, it is necessary to embed the anchor bolt near the surface of the concrete and the results of the test therefore may not be representative of the overall concrete structure. Second, the pull-out forces tend to impose shear and tensile stresses along the potential rupture surface, whereas the actual stresses imposed on a concrete member under load tend to be shear and compressive. Third, the pull-out forces are generally imposed in a direction transverse to the direction of actual critical forces imposed under load. Fourth, a relatively large force is required to pull out the anchor bolt so that a specially fabricated jacking and force measuring device is needed. Such devices tend to be expensive and prone to maladjustment or damage under adverse field conditions. Fifth, the jacking device requires a smooth, flat and hard bearing surface to react against, thus requiring special precautions during casting and construction. Sixth, the anchor bolt must extend perpendicular to the bearing surface, thus requiring precautions to ensure that the bolt is not tipped or dislodged during the pouring and setting of the concrete. Finally, the pull out test may cause a conical piece of material to be broken away at the surface of the structural member which is unsightly and may require repair or filling.

It is the object of the present invention to provide a device and procedure for measuring in situ the compressive strength of concrete structural elements which overcomes and circumvents the various deficiencies and limitations in the prior art.

The present invention provides a device and procedure for measuring the strength of brittle material such as concrete in which a body member is embedded in the material and a measurable turning force, rather than a pulling force, is applied to the body member. Patents Abstracts of Japan, Vol. 6, No. 46 (P-107) (924), 24 March, 1982 discloses a device in accordance with the prior art portion of claim 1 and a method in accordance with the prior art portion of claim 5. As compared therewith the present invention provides, as characterised in claim 1 for the body member to be simply rotatable about its own axis with no special holding means being required, the member being completely embedded in the concrete or other brittle material to be tested. The projections extending outwardly from the body member may be secured thereto or integral therewith.

Torque may conveniently be applied to the body member through, as torque applying means, an elongated rod, one end of which is secured to the body member, the opposite end extending outwardly from a surface of the material. The rod may be releasably secured to the body member or formed integrally therewith, and is typically embedded in the material prior to hardening. A conventional torque wrench is attached to the free end of the rod and carries a device capable of measuring the magnitude of the torque applied to the body member.

To measure the compressive strength of a concrete member, the torque wrench is manually operated to turn the elongated rod in one direction to break the bond between the hardened material and the surface of the rod. The torque required to break this bond may then be measured. The torque wrench is then operated to turn the elongated rod in the opposite direction so that it engages the body member and exerts a turning force thereon which is translated into a compressive force against a portion of the concrete. The torque is increased until either a value corresponding to the lower limit strength of the material is reached or the concrete ruptures. At this point, the elongated rod may be removed and the resulting void filled if desired.

This invention thus provides an improved device and procedure for measuring the strength of concrete structural elements. Strength can be measured at any desired depth and from any accessible exposed or favored surface. The volume of material disposed during measurement is minimal and does not vary with the depth of the location chosen. The critical stresses imposed on the concrete during the test are shear and compressive stresses, which are analogous to the actual stresses encountered under load, and are in the plane of the critical forces imposed under load. Application of the required torque is accomplished manually, is within the capability of average adults, and does not require contact with the surface of the structural member. Finally, any rupturing of the concrete during the test is completely internal and not visually discernible from the surface, and does not significantly affect the post-measurement strength of the structural member.

Further objects, features and advantages of this invention will become apparent from a consideration of the accompanying description, the appended claims, and the following drawing in which:

Fig. 1 is a side view of a concrete structural element, broken away to show the measuring device of the present invention operatively associated therewith;

Fig. 2 is an enlarged side view of the body member and elongated rod of the present invention;

Figs. 3—5 are top sectional views of the body member of this invention, disclosing alternative forms thereof, as seen from substantially the line 3—3 in Fig. 2; and

Figs. 6 and 7 are enlarged side sectional views of the body member of this invention, disclosing alternative forms thereof.

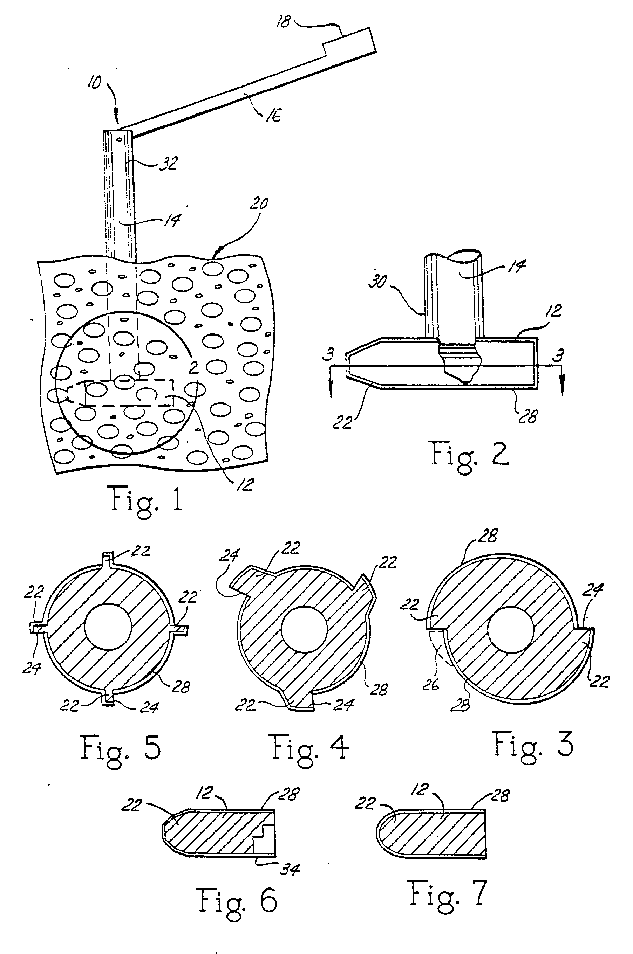

With reference to the drawing, the measuring device of this invention, indicated generally at 10, is shown in Fig. 1 in position to measure the strength of a concrete structural member 20. The device 10 includes a body member 12, an elongated rod 14, and a conventional torque wrench 16 to which a torque measuring device 18 is attached. The strength of the structural member 20 is measured by applying a measured torque to the body member 12, which exerts a compressive force on the concrete, as will appear more fully hereinafter.

As seen in Fig. 2, the body member 12 is preferably thin and disc-like, although the invention is not limited to a body member of any particular size and shape. One or more projections 22 are provided on the body member 12 and extend substantially radially outwardly therefrom. The projections 22 may be secured to the body member 12 or they may be formed integrally therewith. Figs. 3—5 show some of the alternative forms the body member 12 may assume. Each projection 22 provides a reacting surface 24 which is in a plane substantially parallel to the axis of rotation of the body member 12, so that upon application of torque to the body member 12, a compressive force is exerted on the concrete in the area of the projections 22.

In testing the strength of structural members, torque is applied to the body member 12 until a value corresponding to the lower limit strength of the material is achieved or until the material ruptures. When rupture occurs, a piece of material in the shape of a convex cusp breaks away, as indicated at 26 in Fig. 3. A compressible zone 28 is provided on the body member 12 into which the convex cusp 26 can move upon rupture. The zone 28 may, for example, comprise a layer of yieldable resilient material, such as rubber, polyurethane or polyethylene, applied to the body member 12. The layer 28 will yield sufficiently to accommodate the cusp 26 to assure that rupture is unimpeded and readily detectable.

Torque is applied to the body member 12 through the elongated rod 14. The rod 14 has a first end portion 30 which is secured to the body member 12 and an opposite end portion 32 which extends outwardly from a surface of the structural member 20. The torque wrench 16 is secured to the free end 32 of the rod 14. In one embodiment, the rod 14 is releasably secured to the body member 12 so that when the rod 14 is turned in one direction it turns independently of the body member 12, and when it is turned in the opposite direction, it causes the body member 12 to be turned with it. For example, the rod end portion 30 and the body member 12 may be cooperatively threaded (Fig. 2), or the end portion 30 may be provided with a cleat 34 which is engageable with the body member 12 (Fig. 6). In an alternative embodiment, the rod 14 may be fixed to the body member 12 or formed integrally therewith (Fig. 7).

The strength of the structural member 20 is measured as follows. First, the body member 12 and the elongated rod 14 are embedded in the concrete before it hardens. The body member 12 may be embedded at any depth and in any orientation, provided the free end 32 of the rod 14 extends outwardly from any surface of the structural member 20. The surface from which the rod 14 extends need not be smooth, level and planar, nor need it be the top surface of the structural member.

After the concrete has hardened, the torque wrench 16 is attached to the free end 32 of the rod 14 and operated to apply a torque to the rod 14. The amount of torque applied to the rod 14 depends upon the force applied to the end of the torque wrench and the length of the torque wrench. By providing a torque wrench 16 of appropriate length, a large amount of torque can be applied to the rod 14 through a small amount of force on the end of the torque wrench 16. Thus, although a large amount of torque is required in measuring the strength of the material, adults of average size and strength are fully capable of supplying the necessary force.

To exert a compressive force on the concrete, torque tending to turn the rod 14 and the body member 12 in one direction (counterclockwise in Figs. 3—5) is applied. However, it will normally be preferable to first rotate the rod 14 in the opposite direction to break the bond between the concrete and the rod caused by hardening of the concrete. In embodiments of this invention wherein the rod 14 is integral with the body member 12 or is otherwise not releasably secured thereto, the body member 12 will also be rotated in this direction prior to the exertion of compressive force, and consequently, the bond between the concrete and the body member will first be broken. The torque required to break the bond may be measured and used in calculating the strength of the concrete.

Torque is applied in the direction in which compressive force is exerted on the concrete in increasing magnitude until a value corresponding to the desired lower limit strength of the material is achieved or until the concrete ruptures. In embodiments wherein the rod 14 is releasably secured to the body member 12, the rod 14 is then removed from the concrete, leaving a neat cylindrical hole which can readily be filled if desired. In embodiments wherein the rod 14 is not releasably secured to the body member 12, the rod 14 may be cut off at the surface of the structural member. The procedure of this invention thus ensures that no unsightly defects remain in tested structural members and that the strength of structural members is not affected by the test.

The invention provides an improved device and procedure for measuring in situ the strength of concrete structural members. The device exerts combined compressive and shear forces which correspond more precisely to actual forces encountered by structural members under load than do the forces exerted by conventional devices. The device is easier to use, is usable in more locations and orientations, and has less effect on the strength and appearance of structural members than conventional devices.

## Claims

1. A device for measuring the strength of brittle material such as concrete comprising a body member (12) adapted to be inserted into the brittle material (20) and having a reacting surface (24) thereon operable to exert a compressive force on the brittle material in response to the application of a measurable torque applied about the axis of the body member, characterised in that the body member comprises a substantially disc-like member (12) adapted to be embedded in the brittle material with the torque applying means (14) extending at right angles to the plane of the disc to the exterior of the brittle material, the reacting surface (24) being provided on at least one projection (22) extending outwardly from the periphery of the disc so that the reacting surface will exert the compressive force on the brittle material responsive to the application of torque to rotate the disc-like member in the plane of the disc.

2. A device according to claim 1, characterised by a layer (28) of compressible material on at least a portion of the external surfaces of said body member (12) exclusive of said reacting surface (24).

3. A device according to claim 2, characterised in that the radially outer surface of said disc-like member extending in one circumferential direction from said reacting surface (24) meets said reacting surface at the radially inner end thereof, the radially outer surface of said disc-like member extending in the opposite direction from said reacting surface meets said reacting surface at the radially outer end thereof, and said radially outer surface of said disc-like member adjacent said reacting surface at the radially inner and outer ends thereof has said layer (28) of compressible material disposed thereon.

4. A device according to claim 1, 2 or 3, characterised in that the torque applying means includes an elongated rod (14) having one end (30) secured to said body member (12) and the other end (32) extending outwardly from a surface of said brittle material and a torque wrench (16) secured to said elongated rod other end (32) applying a force to said elongated rod tending to turn said rod, and therefore said body member, about the axis of the rod.

5. A method of measuring the compressive strength of brittle material (20) such as concrete comprising the steps of inserting in the material (20) to be tested a body member (12) having a reacting surface (24), applying a torque to said body member (12) tending to turn said body member (12) in one direction causing said reacting surface (24) to exert a compressive force on said brittle material (20), and measuring said torque, characterised in that the body member (12) is of a device as claimed in any preceding claim.

6. A method according to claim 5, characterised in that said body member is first rotated in the direction opposite to said one direction.

7. A method according to claim 6, characterised in that the torque required to rotate said body member in said opposite direction is also measured.

8. A method according to claim 5, 6 or 7, characterised in that torque is applied up to a predetermined value corresponding to the required strength of the material.

9. A method according to claim 5, 6 or 7, characterised in that torque of an increasing magnitude is applied until the material ruptures.

## Patentansprüche

1. Vorrichtung zur Messung der Festigkeit von bröckligem Material wie Beton, mit einem Körperelement (12), das zum Einsetzen in das bröcklige Material (20) gestaltet ist und eine Reaktionsfläche (24) aufweist, die eine Druckkraft auf das bröcklige Material in Abhängigkeit von der Anwendung eines meßbaren Drehmoments aufbringen kann, das an der Achse des Körperelements ansetzt, dadurch gekennzeichnet, daß das Körperelement ein im wesentlichen scheibenförmiges Element (12) ist, das in das bröcklige Material einbringbar ist, wobei sich das Drehmoment aufwendende Mittel (14) im rechten Winkel zur Ebene der Scheibe zum Äußeren des bröckligen Materials erstreckt, daß die Reaktionsfläche (24) mit mindestens einem Fortsatz (22) versehen ist, der sich vom Umfang der Scheibe nach außen erstreckt, so daß die Reaktionsfläche die Druckkraft auf das bröcklige Material in Abhängigkeit von der Anwendung von Drehmoment zum Drehen des scheibenförmigen Elements in der Scheibenebene ausübt.

2. Vorrichtung nach Anspruch 1, gekennzeichnet durch eine Schicht (28) aus zusammendrückbarem Material auf zumindest einem Teil der äußeren Flächen des Körperelements (12) mit Ausnahme der Reaktionsfläche (24).

3. Vorrichtung nach Anspruch 2, dadurch gekennzeichnet, daß die radiale Außenfläche des scheibenförmigen Elements, die sich in einer Umfangsrichtung von der Reaktionsfläche (24) erstreckt, die Reaktionsfläche am radialen inneren Ende davon trifft, daß die radiale äußere Fläche des scheibenförmigen Elements, die sich in entgegengesetzter Richtung zu der Reaktionsfläche erstreckt, die Reaktionsfläche an deren radialen äußeren Ende trifft, und daß die radiale äußere Fläche des scheibenförmigen Elements in der Nähe der Reaktionsfläche an deren radialen inneren und äußeren Enden die Schicht (28) aus zusammendrückbarem Material darauf aufgebracht hat.

4. Vorrichtung nach Anspruch 1, 2 oder 3, dadurch gekennzeichnet, daß das Drehmoment anlegende Mittel eine längliche Stange (14) aufweist, die mit einem Ende (30) an dem Körperelement (12) befestigt ist und deren anderes Ende (32) sich von einer Oberfläche des brüchigen Materials nach außen erstreckt, und daß ein Drehmomentenschlüssel (16) an dem anderen Ende (32) der länglichen Stange befestigt ist und eine Kraft auf die längliche Stange aufbringt, die dazu neigt, die Stange und dadurch das Körperelement um die Achse der Stange zu drehen.

5. Verfahren zum Messen der Druckfestigkeit von brüchigem Material (20) wie Beton, mit den Schritten Einsetzten eines Körperelements (12) mit einer Reaktionsfläche (24) in das zu prüfende Material (20), Anwenden eines Drehmoments auf das Körperelement (12), welches dazu neigt, das Körperelement (12) in eine Richtung zu drehen, was die Reaktionsfläche (24) veranlaßt, eine Druckkraft auf das brüchige Material (20) auszuüben, und Messen des Drehmoments, dadurch gekennzeichnet, daß das Körperelement (12) eine Vorrichtung nach einem der vorhergehenden Ansprüche ist.

6. Verfahren nach Anspruch 5, dadurch gekennzeichnet, daß das Körperelement zuerst in Richtung entgegengesetzt zu der einen Richtung gedreht wird.

7. Verfahren nach Anspruch 6, dadurch gekennzeichnet, daß das zum Drehen des Körperelements in die entgegengesetzte Richtung erforderliche Drehmoment ebenfalls gemessen wird.

8. Verfahren nach Anspruch 5, 6 oder 7, dadurch gekennzeichnet, daß ein Drehmoment bis zu einem vorgegebenen Wert entsprechend der erforderlichen Festigkeit des Materials angewendet wird.

9. Verfahren nach Anspruch 5, 6 oder 7, dadurch gekennzeichnet, daß ein Drehmoment mit einer zunehmenden Größe angelegt wird, bis das Material bricht.

**Revendications**

1. Dispositif permettant de mesurer la résistance d'un matériau cassant tel que le béton, comprenant un corps pouvant s'encastrer dans le matériau cassant (20) et possédant sur celui-ci une surface de réaction (24) pouvant exercer une force de compression sur le matériau friable, en réponse à un couple mesurable appliquée sur l'axe du corps, caractérisé par le fait que le corps comprend une pièce ayant pratiquement la forme d'un disque (12) pouvant s'encastrer dans le matériau cassant, le moyen d'application du couple (14) se projetant, perpendiculairement au plan du disque, vers l'extérieur du matériau cassant, la surface de réaction (24) étant pourvue d'au moins une saillie (22) se projetant à partir de la circonférence du disque vers l'extérieur, de manière à ce que la surface de réaction puisse exercer la force de compression sur le matériau cassant en réponse à l'application du couple, afin de faire tourner la pièce en forme de disque dans le plan du disque.

2. Dispositif selon la revendication 1, caractérisé par une couche (28) de matériau compressible sur au moins une partie des surfaces externes dudit corps (12), à l'exclusion de ladite surface de réaction (24).

3. Dispositif selon la revendication 2, caractérisé par le fait que la surface radiale extérieure de ladite pièce en forme de disque, se projetant à partir de ladite surface de réaction (24) dans un des sens de la circonférence, rencontre ladite surface de réaction à l'extrémité radiale intérieure du disque, la surface radiale extérieure de ladite pièce en forme de disque se projetant dans la direction opposée à ladite surface de réaction, rencontre ladite surface de réaction à l'extrémité radiale extérieure du disque. Ladite surface radiale extérieure de ladite pièce en forme de disque, jouxtant ladite surface de réaction aux extrémités radiales extérieure et intérieure de celle-là est recouverte d'une couche (28) de matériau compressible.

4. Dispositif selon les revendications 1, 2 ou 3, caractérisé par le fait que le moyen d'application du couple comprend une barre oblongue (14) dont une extrémité (30) est fixée audit corps (12), et dont l'autre extrémité (32) se projette vers l'extérieur à partir d'une des surfaces dudit matériau cassant, ainsi qu'une clef dynamométrique (16) fixée sur l'autre extrémité de ladite barre oblongue (32), appliquant une force sur celle-ci et tendant à faire tourner ladite barre et par conséquent, ledit corps autour de l'axe de la barre.

5. Procédé pour mesurer la résitance à la compression d'un matériau cassant (20), tel que le béton, comprenant les étapes consistant à insérer, dans le matériau (20) devant être testé, un corps (12) ayant une surface de réaction (24), à appliquer un couple sur ledit corps (12), tendant à faire tournir ledit corps (12) dans un sens, provoquant une force de compression de ladite surface de réaction (24) sur ledit matériau cassant (20); et à mesurer ledit couple, caractérisé par le fait que le corps (12) fait partie du dispositif, comme l'indiquent toutes les revendications précédentes.

6. Procédé, selon la revendication 5, caractérisé par le fait que ledit corps pivote d'abord dans le sens opposé audit sens.

7. Procédé, selon la revendication 6, caractérisé par le fait qu'on mesure également le couple nécessaire pour faire tourner ledit corps dans ledit sens opposé.

8. Procédé, selon les revendications 5, 6, ou 7, caractérisé par le fait que le couple est appliqué jusqu'à une valeur prédéterminée, correspondant à la résistance requise du matériau.

9. Procédé, selon les revendications 5, 6, ou 7, caractérisé par le fait qu'un couple d'une intensité croissante est appliquée jusqu'à la rupture du matériau.

Fig. 1

Fig. 2

Fig. 5

Fig. 4

Fig. 3

Fig. 6

Fig. 7